# FASCICULE DE BREVET EUROPEEN

(11) **EP 2 659 835 B1**
(45) Date de publication et mention de la délivrance du brevet: **12.11.2014**
(21) Numéro de dépôt: 13305490.8
(22) Date de dépôt: 16.04.2013
(51) Int. Cl.: A61B 5/22

(54) **Dispositif pour quantifier l'indépendance des doigts**
Vorrichtung zur Quantifizierung der Unabhängigkeit der Finger
Device for quantifying finger independence

(30) Priorité: 03.05.2012 FR 1254063
(43) Date de publication de la demande: 06.11.2013
(73) Titulaire: Sensix, 86000 Poitiers (FR); Université Paris Descartes, 75270 Paris Cedex 06 (FR)
(72) Inventeur: Boucher, Mathieu, 86000 POITIERS (FR); Maier, Marc, 75010 PARIS (FR); Lindberg, Pavel, 75011 PARIS (FR)
(74) Mandataire: Cabinet Laurent & Charras

(56) Documents cités:
- DE-A1- 3 929 636
- JP-A- 10 057 357
- US-A- 3 738 651
- US-A- 5 299 457
- US-A- 5 690 585
- US-A1- 2009 318 269
- SANTELLO M ET AL: "Control of multidigit grasping in Parkinson's disease: effect of object property predictability", EXPERIMENTAL NEUROLOGY, ACADEMIC PRESS, NEW YORK, NY, US, vol. 187, no. 2, 1 juin 2004 (2004-06-01), pages 517-528, XP004620628, ISSN: 0014-4886, DOI: 10.1016/J.EXPNEUROL.2004.02.016

## Description

L'invention se rattache au secteur technique des appareils, instruments et dispositifs qui permettent de détecter et de mesurer le mouvement des doigts, et ce, dans le cadre d'études de pathologies neurologiques, myologiques ou arthrologiques pour le diagnostic, le pronostic clinique et la rééducation d'un ou de plusieurs doigt(s).

L'invention se rattache à des appareils et dispositifs de mesures qui permettent d'établir sous l'action de forces la capacité de mouvement de chacun des doigts des mains d'une personne et qui permettent aussi la musculation et rééducation des doigts.

De nombreuses études ont ainsi été développées pour mesurer la dextérité des doigts d'une main, la capacité de mouvement de chaque doigt, et ce, dans le cadre de l'exercice d'une force de pression. Ces appareils ou dispositifs sont essentiellement utilisés à des fins thérapeutiques et médicales et de musculation en rééducation. La dextérité manuelle est souvent affectée par des pathologies neurologiques (accident vasculaire cérébral, paralysie cérébrale, myélopathie cervico-arthrosique et autres) ou des pathologies orthopédiques et nécessite une quantification pour tout diagnostic et pronostic clinique ou une éventuelle rééducation des doigts.

Au titre de l'art antérieur, on peut par exemple citer les dispositifs ou appareils décrits dans les brevets EP 2218401, EP 1567056, EP 1578248, US 6537075, US 5317916, EP 495461, US 5157970, US 5170633, US 5690585, US 5147256, US 5431611, SU 1660709, US 3738651, US 1796216.

Certains des dispositifs et appareils ont aussi pour objectifs de permettre simplement une musculation des doigts.

De manière générale, ces dispositifs ou appareils comprennent des touches au nombre de 4 associées à des capteurs qui permettent d'effectuer la mesure. Des moyens électroniques sont associés à chacun des dispositifs pour permettre une transmission et une gestion des données qui ont été recueillies pour les analyses appropriées.

Les dispositifs ou appareils, qui ont été développés et existants sur le marché, restent d'une conception complexe, coûteuse, et difficilement utilisable par les patients et sont mal adaptés aux capacités motrices des utilisateurs. Les appareils connus sur le marché sont généralement positionnés statiquement sur un plan support tel qu'une table et ne sont pas manipulables dans la main. Certains appareils sont même connectés à des robots avec un coût élevé et une maniabilité très réduite.

La démarche du Demandeur a été de concevoir un dispositif simplifié permettant de mesurer et de quantifier l'indépendance des doigts à la fois au niveau du déplacement (cinématique) et au niveau de la force exercée (dynamique). En d'autres termes, le but recherché selon l'invention était de proposer un dispositif d'une grande facilité d'utilisation, d'une grande adaptabilité, pour effectuer des mesures tant en régime isométrique qu'en régime non isométrique.

La démarche du Demandeur a aussi été de vouloir concevoir un dispositif permettant de mesurer l'indépendance des doigts et qui soit maniable très facilement par l'opérateur ou utilisateur, et pouvant être tenu dans la paume de la main et manipulé par les doigts de l'utilisateur.

La démarche du Demandeur a aussi été de créer un appareil qui permette l'analyse des forces dans des tâches différentes, et non limitativement en relation à la contraction d'un doigt en isolation qui amène à une contraction involontaire des doigts voisins.

En d'autres termes, le dispositif selon l'invention a été conçu pour avoir de plus larges applications dans les différentes mesures et contrôles effectués tout en restant simple et maniable.

Un autre objectif selon l'invention a été de concevoir le dispositif pour qu'il permette un ajustement des besoins d'analyse, soit en régime isométrique uniquement, soit en régime non isométrique, soit dans un régime mixte isométrique-non isométrique.

C'est donc par rapport à l'ensemble de ces objectifs et buts à atteindre que le Demandeur a mis au point le nouveau dispositif de quantification de l'indépendance des doigts, objet de la présente invention.

Selon une première caractéristique, le dispositif pour quantifier l'indépendance des doigts, comprenant une pluralité de pistons insérés dans une structure réceptrice et permettant un appui digital des doigts d'une main à l'exception du pouce et soumis à un déplacement à l'encontre de moyens de rappels pour être en contact avec des capteurs de force transmettant des informations à une unité informatique d'enregistrement, est remarquable en ce que le module est établi sous forme d'un module destiné à être manipulé par un patient utilisateur et tenu dans la paume de la main, le dit module comprenant une structure de réception de sous-ensembles, chaque sous-ensemble étant disposé verticalement et comprenant un manchon à position fixe creux intérieurement pour le passage d'un piston, le dit piston étant monté à libre coulissement dans le manchon et étant creux et fileté intérieurement pour la réception d'une vis de réglage du piston par rapport à un élément capteur disposé sous-jacent, et ce à l'encontre d'un moyen de rappel et de compression, et en ce que la position du piston préréglé par la vis permet d'effectuer des mesures non isométriques lors du déplacement du piston, et des mesures isométriques lors du blocage en position du piston réalisé soit par la compression maximum du moyen de rappel et ainsi le contact entre le piston et le disque monté au dessus du capteur soit par le vissage de la vis de réglage jusqu'à obtenir le contact entre le bout plat de la vis et la face supérieure d'un disque en relation avec le dit capteur.

Ces caractéristiques et d'autres encore ressortiront bien de la suite de la description.

Pour fixer l'objet de l'invention illustré d'une manière non limitative aux figures des dessins où :

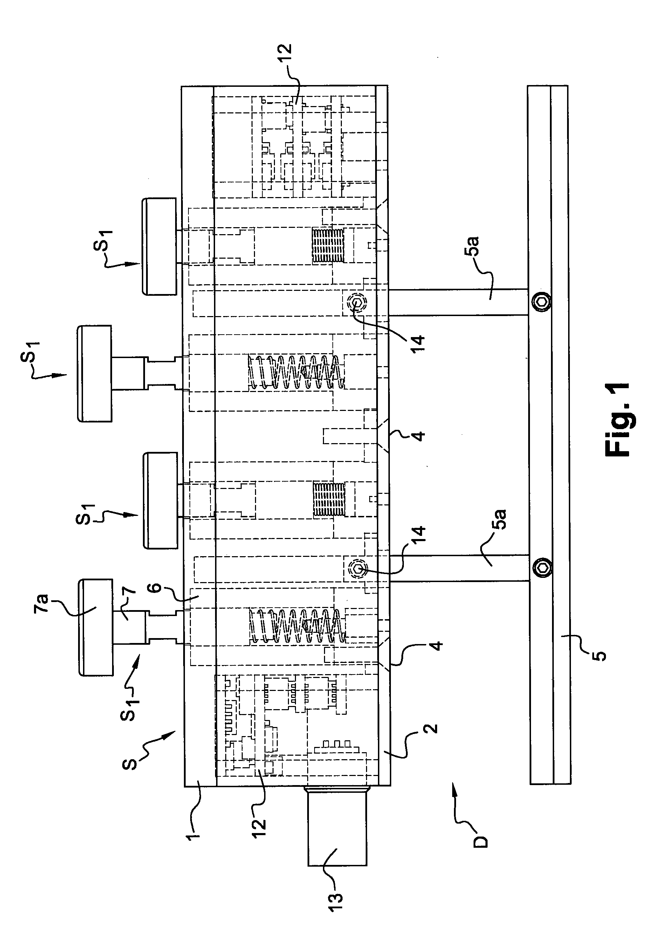

La figure 1 est une vue de face du dispositif selon l'invention incluant quatre pistons pour être sollicités par les doigts de la main à l'exception du pouce, et étant tenu dans la paume de la main,

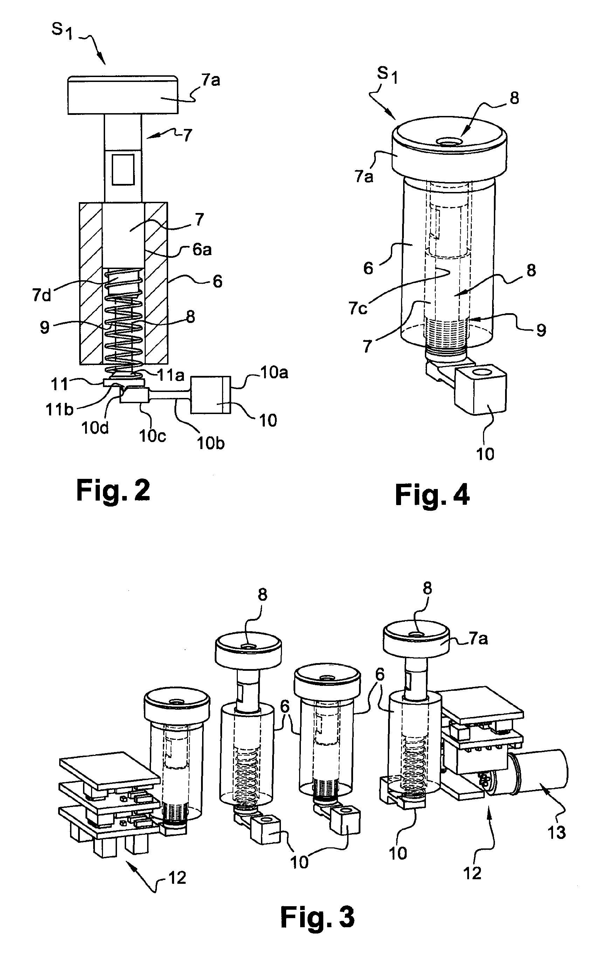

La figure 2 est une vue d'un sous-ensemble comprenant un piston,
La figure 3 est une vue éclatée avant montage illustrant les différents sous-ensembles avec pistons constituant le dispositif,
La figure 4 est une vue complémentaire à la figure 2 dans une position différente du piston,

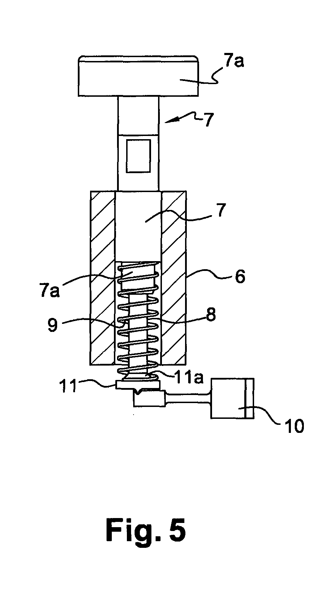

La figure 5 est une vue complémentaire à la figure 2 d'un sous-ensemble comprenant un piston et une vis.

Afin de rendre plus concret l'objet de l'invention, on le décrit maintenant d'une manière non limitative illustré aux figures des dessins.

Le dispositif de mesure de l'indépendance des doigts est référencé dans son ensemble par (D). Il est établi sous la forme d'un module pouvant être manipulé par un patient utilisateur et pouvant être tenu dans la paume de la main. Il comprend inséré dans une structure de réception (S) au moins un et de préférence quatre sous-ensembles correspondant à chacun des doigts de la main à l'exception du pouce et pouvant être manipulé pour permettre l'établissement d'une mesure de force à l'aide de capteurs disposés en partie basse de chaque sous-ensemble. Plus spécifiquement, la structure de réception (S) comprend un corps (1) et une plaque horizontale de protection (2). Le corps permet la réception et le positionnement de chacun des sous-ensembles (S1) et des capteurs. La plaque de protection est fixée sur le corps à l'aide de moyens de vissage (4) afin d'éliminer tout risque de contact mécanique entre l'utilisateur et les sous-ensembles (S1) ainsi que les capteurs (10) et l'électronique embarquée (12).

Le dispositif comprend en outre un plan d'appui (5) pouvant être tenu dans la paume de la main et établi sous forme de plaque qui présente des bras (5.a) associés et solidarisés aux montants fixes du corps (1) à l'aide de moyens de vissage (14). Le plan d'appui (5) peut glisser grâce à ses quatre bras (5.a) afin d'adapter la taille dispositif (D) à la paume de la main de l'utilisateur.

Chacun des sous-ensembles (S1) est disposé verticalement et comprend un manchon (6) à position fixe creux intérieurement pour le passage d'un piston (7). Chaque manchon est de relative grande hauteur et est inséré dans le logement cylindrique du corps (1) lui correspondant. Chaque piston comprend une tête de piston (7.a) débordante du manchon récepteur, la dite tête se prolongeant par un corps cylindrique (7.b) pénétrant et étant guidée dans le trou (6.a) intérieur formé sur chaque manchon. Ce corps présente dans sa partie basse une forme épaulée (7.d). Le corps du piston est ainsi monté à libre coulissement à l'intérieur du dit manchon. Le piston dans sa partie corps est creux intérieurement en étant fileté sur tout ou partie de sa longueur (7.c). Ce filetage est également établi dans la tête du piston (7.a) pour permettre le passage et l'introduction d'une vis (8) qui pénètre par le centre de la tête du piston. Cette vis (8) est de grande longueur, sans tête et avec un bout plat. La forme épaulée du piston (7.d) permet de positionner un moyen de rappel et de compression (9) du type ressort. Ce dernier est donc centré dans le manchon autour de la partie cylindrique inférieure du corps du piston (7.d). Sous-jacent à chacun des manchons est disposé un élément capteur (10) de construction spécifique. Ce dernier comprend en effet une base (10.a) sous-forme de cube ou parallépipédique fixée sur le corps (1) de toute manière appropriée. Cette base se prolonge vers le piston par un bras (10.b) positionné horizontalement et dont l'extrémité est agencée avec une plateforme (10.c) en forme de parallélépipède et présentant une forme en relief (10.d) creuse dans l'épaisseur de la dite plateforme (10.c). Celle-ci se situe sensiblement en-dessous du manchon et elle reçoit un disque (11) qui est conformé avec un relief (11.a) permettant le centrage et l'appui de la seconde extrémité du moyen de rappel (9). Le disque (11) présente une empreinte (11.b) destinée à être en regard de la forme en relief creuse (10.d) établie sur la plateforme (10.c) du capteur. Il y a donc un contact établi entre le disque (11) et la plateforme (10.c) et en fonction de l'effort exercé sur le piston considéré se produit une déformation au niveau du bras de levier (10.b). Une jauge de contrainte et de déformation est disposée sur chacun des bras en étant reliée à des moyens (12) de conditionnement électronique. La structure reçoit également un connecteur (13) et permet la sortie des signaux analogiques qui ont été générés par les différentes jauges de contrainte associées indirectement à chacun des pistons. L'ensemble des capteurs est ainsi relié à une unité informatique permettant d'analyser, comparer et donner les informations nécessaires.

Ainsi qu'il apparaît aux dessins, les actions sont effectuées par le patient utilisateur sur chacun des pistons ou tête de piston d'une manière libre et sans attache particulière. L'appui digital sur chacun des pistons va provoquer selon le positionnement initial du piston une compression ou non de chacun des moyens de rappel et de compression (9).

En effet, dans l'hypothèse où seule une mesure isométrique est requise, il est nécessaire de bloquer en position chacun des pistons en position haute dans la structure (S1), c'est-à-dire avec une compression minimale du moyen de rappel correspondant. Cela s'effectue par l'action de vissage de la vis sans tête qui est déplacé vers le bas par rapport à son piston récepteur. Le vissage s'effectue par tous moyens appropriés du type tournevis. L'appui est donc effectué au maximum sur la partie en forme de parallélépipède du capteur. Les vis de blocage permettent d'éliminer le débattement du piston et ce dernier est en contact direct avec le capteur par l'intermédiaire de la vis. Dans ce cas, on ne mesure que la force de pression du doigt de l'utilisateur grâce à la jauge de contrainte associée à ce capteur. On est en situation isométrique.

Grâce à l'invention, on peut aussi effectuer des mesures non isométriques en combinant le déplacement cinématique du piston avec la force énergétique générée par l'appui du moyen de rappel sur le capteur. Dans ce cas, chacune des vis est remontée à l'intérieur du piston et de la tête du piston de sorte que le ressort (9) n'est plus comprimé, et que le piston est en position remontée dans son manchon récepteur. Le piston est relié au capteur via le ressort pendant tout le débattement du piston, puis en contact direct sur le capteur via la base inférieure du piston. Le capteur mesure donc à la fois l'énergie transmise par le déplacement du piston, et ensuite la compression du ressort générée par l'appui des doigts du patient utilisateur. Le passage de la mesure non isométrique à la mesure isométrique s'effectue de la manière suivante lors du débattement ou déplacement du piston. Lorsque le patient exerce une force supérieure à F min, cela provoque le déplacement du piston. Le piston est déplacé à son amplitude maximale lorsque la force exercée est égale à F max. Ensuite, le piston étant en contact direct avec le capteur, le dispositif fonctionne en isométrique. Les valeurs F min et F max sont des valeurs seuils à définir selon le type de déficience à traiter ou selon les diagnostics à faire.

Cette disposition particulière offre un très gros intérêt en ce qu'il permet une mesure beaucoup plus complète et indépendante des doigts du patient utilisateur. A titre indicatif, la taille du dispositif est de l'ordre de 110 mm par 30 mm, avec une hauteur réglable entre 53,5 à 75 mm. Les efforts digitaux sont appliqués sur des pistons à 10 mm de débattement. La raideur de chaque ressort de précision est de 0,09 N/mm. L'enfoncement maximal du piston est alors obtenu sous une charge de 1N et la force nécessaire au déplacement initial du piston est de 0,1N. Dans cette gamme, la mesure est non isométrique. La mesure des efforts digitaux est effectuée par les quatre capteurs mono-axiaux situés sous chaque piston. L'étendue de la mesure est de 0 - 10 N. Le conditionnement du signal et la précision des capteurs permettent de délivrer une résolution de 0,01 N. Pour une force > 1 N, la force est mesurée en isométrique sans déplacement du piston.

Les avantages ressortent bien de l'invention.

On souligne la simplicité du dispositif, sa grande maniabilité, et également sa capacité à effectuer différents types de mesures par un réglage simple et rapide de la position de chacun des pistons à l'aide des vis de réglage. La position du point d'application de la force sur le capteur est ainsi constante, la mesure de la flexion de la lame du capteur permet alors de déduire avec précision cette dite force.

Il est possible aussi par ce biais de faire des mesures différenciées avec des positions respectives de pistons différentes pour chaque doigt. L'invention permet de quantifier l'indépendance des doigts à la fois au niveau du déplacement (cinématique) et au niveau de sa force (dynamique). Le capteur peut être utilisé en régime isométrique simplement.

## Revendications

1. Dispositif pour quantifier l'indépendance des doigts, comprenant une pluralité de pistons insérés dans une structure réceptrice et permettant un appui digital des doigts d'une main à l'exception du pouce et soumis à un déplacement à l'encontre de moyens de rappels pour être en contact avec des capteurs de force transmettant des informations à une unité informatique d'enregistrement, **caractérisé en ce que** le module est établi sous forme d'un module destiné à être manipulé par un patient utilisateur et tenu dans la paume de la main, le dit module comprenant une structure de réception (S) de sous-ensembles (S1), chaque sous-ensemble étant disposé verticalement et comprenant un manchon (6) à position fixe creux intérieurement pour le passage d'un piston (7), le dit piston étant monté à libre coulissement dans le manchon et étant creux et fileté intérieurement pour la réception d'une vis (8) de réglage du piston par rapport à un élément capteur (10) disposé sous-jacent, et ce à l'encontre d'un moyen de rappel et de compression (9), **et en ce que** la position du piston préréglé par la vis (8) permet d'effectuer des mesures non isométriques lors du déplacement du piston, et des mesures isométriques lors du blocage en position du piston réalisé soit par la compression maximum du moyen de rappel (9) et ainsi le contact entre le piston (7) et le disque (11) monté au dessus du capteur (10) soit par le vissage de la vis de réglage jusqu'à obtenir le contact entre le bout plat de la vis et la face supérieure d'un disque (11) en relation avec le dit capteur (10).

2. Dispositif selon la revendication 1, **caractérisé en ce que** la structure de réception comprend un corps (1) et une plaque (2) horizontale de protection, le corps permettant la réception et le positionnement de chacun des sous-ensembles (S1) et des capteurs, la liaison entre la plaque de protection et le corps se faisant par vissage (4), **et en ce qu'**il comprend un plan d'appui (5) tenu dans la paume de la main et établi sous forme de plaque avec des bras entretoisés (5a) fixés à la structure (S).

3. Dispositif selon l'une quelconque des revendications 1 et 2, **caractérisé en ce que** chaque piston comprend une tête (7a) débordante du manchon récepteur, la dite tête se prolongeant par un corps cylindrique (7b) guidé dans le manchon (6) à libre coulissement, le dit piston étant fileté dans son corps sur tout ou partie de sa longueur (7c) pour l'introduction de la vis (8), le dit corps présente également une forme épaulée dans sa partie inférieure (7.d) autorisant le positionnement d'un moyen de rappel et de compression (9), **et en ce que** sous-jacents à chacun des manchons sont disposés des capteurs (10) agencés avec des disques (11) permettant la réception de l'autre extrémité du moyen de rappel, les dits capteurs étant sollicités soit uniquement par le moyen de rappel lorsque le piston est en déplacement dans le manchon (6), soit par le moyen de rappel et la force de contact entre le piston et le disque (11) lorsque le piston est en butée sur le disque, soit enfin par la force de contact entre l'extrémité de la vis et le disque(11) dans la position isométrique.

4. Dispositif selon la revendication 3, **caractérisé en ce que** chaque capteur (10) présente une base (10a) avec en prolongement un bras (10b) dont l'extrémité est agencée avec une plateforme (10) présentant un relief (10d) creux, le disque (11) présentant un relief (11a) pour le centrage et l'appui du ressort (9) et une empreinte (11b) pour être en contact avec le relief (10d), **et en ce que** la position du point d'application de la force sur le capteur est ainsi constante, la mesure de la flexion de la lame du capteur permettant alors de déduire avec précision cette dite force.

5. Dispositif selon l'une quelconque des revendications 1 et 3, **caractérisé en ce que** les vis (8) de grande longueur sont sans tête et avec un bout plat.

## Patentansprüche

1. Vorrichtung zum Quantifizieren der Unabhängigkeit von Fingern, die mehrere Kolben umfasst, die in eine Aufnahmestruktur eingesetzt sind und eine Abstützung der Finger einer Hand mit Ausnahme des Daumens ermöglichen und einer Verlagerung entgegen Rückstellmitteln unterworfen werden, um mit Kraftsensoren in Kontakt zu gelangen, die Informationen an eine Datenaufzeichnungseinheit senden, **dadurch gekennzeichnet, dass** das Modul in Form eines Moduls gebildet ist, das dazu bestimmt ist, durch einen Anwender und Patienten manipuliert zu werden und auf der Handfläche der Hand gehalten zu werden, wobei das Modul eine Struktur (S) für die Aufnahme von Unteranordnungen (S1) umfasst, wobei jede Unteranordnung vertikal angeordnet ist und eine fest positionierte innen hohle Muffe (6) für den Durchgang eines Kolbens (7) umfasst, wobei der Kolben frei gleitend in der Muffe montiert ist und hohl ist und ein Innengewinde aufweist, um eine Schraube (8) für die Einstellung des Kolbens in Bezug auf ein darunter angeordnetes Sensorelement entgegen einem Rückstell- und Kompressionsmittel (9) aufzunehmen, und dass die Position des Kolbens, die im Voraus durch die Schraube (8) eingestellt wird, ermöglicht, nicht isometrische Messungen, wenn der Kolben verlagert wird, und isometrische Messungen, wenn der Kolben in seiner Position blockiert ist, was entweder durch die maximale Kompression des Rückstellmittels (9) und somit den Kontakt zwischen dem Kolben (7) und der über dem Sensor (10) montierten Scheibe (11) oder durch das Einschrauben der Einstellschraube, bis der Kontakt zwischen dem flachen Ende der Schraube und der oberen Fläche einer Scheibe (11) in Bezug auf den Sensor (10) erreicht ist, verwirklicht wird, auszuführen.

2. Vorrichtung nach Anspruch 1, **dadurch gekennzeichnet, dass** die Aufnahmestruktur einen Körper (1) und eine horizontale Schutzplatte (2) umfasst, wobei der Körper die Aufnahme und die Positionierung jeder der Unteranordnungen (S1) und der Sensoren ermöglicht, wobei die Verbindung zwischen der Schutzplatte und dem Körper durch Verschrauben (4) erfolgt, und dass sie eine Abstützebene (5) umfasst, die auf der Handfläche gehalten wird und in Form einer Platte gebildet ist, die Abstandshalterarme (5a), die an der Struktur (S) befestigt sind, aufweist.

3. Vorrichtung nach einem der Ansprüche 1 und 2, **dadurch gekennzeichnet, dass** jeder Kolben einen über die Aufnahmemuffe vorstehenden Kopf (7a) umfasst, wobei der Kopf durch einen zylindrischen Körper (7b) verlängert ist, der in der Muffe (6) frei gleitend geführt wird, wobei der Kolben in seinem Körper auf seiner gesamten Länge (7c) oder auf einem Teil hiervon ein Gewinde aufweist, um die Schraube (4) einzuführen, wobei der Körper außerdem in seinem unteren Teil (7d) eine Schulterform aufweist, die die Positionierung eines Rückstell- und Kompressionsmittels (9) erlaubt, und dass unter jeder der Muffen Sensoren (10) angeordnet sind, die mit Scheiben (11) versehen sind, die die Aufnahme des anderen Endes des Rückstellmittels ermöglichen, wobei die Sensoren entweder nur durch das Rückstellmittel eingeklemmt sind, wenn der Kolben in der Muffe (6) verlagert wird, oder durch das Rückstellmittel und die Kontaktkraft zwischen dem Kolben und der Scheibe (11) eingeklemmt sind, wenn der Kolben an der Scheibe anliegt, oder am Ende durch die Kontaktkraft zwischen dem Ende der Schraube und der Scheibe (11) in der isometrischen Position eingeklemmt sind.

4. Vorrichtung nach Anspruch 3, **dadurch gekennzeichnet, dass** jeder Sensor (10) eine Basis (10a) mit einem Arm (10b) in der Verlängerung aufweist, wovon ein Ende mit einer Plattform (10) ausgebildet ist, die ein Hohlrelief (10d) aufweist, wobei die Scheibe (11) ein Relief (11a) aufweist, um die Feder (9) zu zentrieren und abzustützen, und eine Prägung (11b) für den Kontakt mit dem Relief (10d) aufweist, und dass die Position des Ausübungspunkts der Kraft auf den Sensor somit konstant ist, wobei die Messung der Biegung des Plättchens des Sensors dann die präzise Ableitung dieser Kraft ermöglicht.

5. Vorrichtung nach einem der Ansprüche 1 bis 3, **dadurch gekennzeichnet, dass** die Schrauben (8) mit großer Länge keinen Kopf und ein flaches Ende besitzen.

## Claims

1. Device for quantifying the independence of the fingers, incorporating a set of pistons inserted in an accommodating structure and allowing the said pistons to be pressed by the fingers of a hand, except using the thumb, subject to a movement against a return system that brings it into contact with force sensors transmitting information items to an information-recording device, **characterized by** the fact that the module is implemented as a module designed to be handled by a user patient and held in the palm of the hand, with the said module incorporating a reception structure (S) for subassemblies (S1), with each subassembly being positioned vertically, and including a sleeve (6) in fixed position that is internally hollow for the passage of a piston (7), with the said piston being mounted so as to slide freely within the sleeve, and being hollow and threaded internally to accommodate a screw (8) for the adjustment of the piston in relation to a sensor component (10) positioned underneath, against a return and compression system (9), and by the fact that the position of the piston having been previously-adjusted by the screw (8) enables non-isometric measurements during the movement of the piston, and isometric measurements during the locking in position of the piston performed either by maximum compression of the return system (9), and the contact between the piston (7) and the disc (11) mounted above the sensor (10), or through the screwing of the adjustment screw until the achievement of contact between the flat end of the screw and the upper face of a disc (11) in relation to the said sensor (10).

2. Device in accordance with claim 1, **characterized by** the fact that the accommodating structure incorporates a body (1) and a horizontal protection plate (2), with the body allowing the accommodation and positioning of each of the subassemblies (S1) and the sensors, with the connection between the protection plate and the body being accomplished by screwing (4), and by the fact that it incorporates a bearing surface (5) held in the palm of the hand, implemented as a plate with with spaced arms (5a) secured to the structure (S).

3. Axle in accordance with either of claims 1 or 2, **characterized by** the fact that each piston incorporates a head (7a), protruding from the accommodating sleeve, with the said head being extended by a cylindrical body (7b) guided within the free-sliding sleeve (6), with the said piston being threaded within its body over all or part of its length (7c) for the insertion of the screw (8); the said body also incorporates a rimmed form at its bottom part (7d) allowing the positioning of a return and compression system (9), and by the fact that underneath each of the sleeves there are sensors (10) fitted with discs (11) allowing the reception of the other end of the return system, with the said sensors being placed under stress either only when the piston is in movement within the sleeve (6), or by the return system and the force of contact between the piston and the disc (11) when the disc is bearing against the disc, or by the force of contact between the end of the screw and the disc (11) in the isometric position.

4. Device in accordance with claim 3, **characterized by** the fact that each sensor (10) has a base (10a) protruding from an arm (10b) of which the end is fitted with a platform (10) having a hollow relief (10d), with the disc (11) having relief (11a) for the centering and pressure of the spring (9) and an imprint (11b) to be in contact with the relief (10d), and by the fact that the position of the point of application of the force on the sensor is therefore constant, with the measurement of the flexing of the blade of the sensor allowing one to deduce the said force with accuracy.

5. Device in accordance with either of claims 1 and 3, **characterized by** the fact that the long screw (8) is headless and has a flat end.
